# EUROPEAN PATENT SPECIFICATION

(11) **EP 4 205 717 B1**
(45) Date of publication and mention of the grant of the patent: **11.11.2026**
(21) Application number: 21861009.5
(22) Date of filing: 12.07.2021
(51) Int. Cl.: A61F 13/534, A61F 13/535

(54) **ABSORBENT ARTICLE**
ABSORBIERENDER ARTIKEL
ARTICLE ABSORBANT

(30) Priority: 25.08.2020 JP 2020142059
(43) Date of publication of application: 05.07.2023
(73) Proprietor: Kao Corporation, Chuo-ku Tokyo 103-8210 (JP)
(72) Inventor: SUZUKI, Takeshi, Haga-gun, Tochigi 321-3497 (JP); TOMITA, Mina, Haga-gun, Tochigi 321-3497 (JP); ANDO, Eigo, Haga-gun, Tochigi 321-3497 (JP)
(74) Representative: Vossius & Partner Patentanwälte Rechtsanwälte mbB
(86) International application number: PCT/JP2021/026125
(87) International publication number: WO 2022/044579

(56) References cited:
- EP-A1- 2 901 992
- JP-A- 2015 518 773
- JP-A- 2016 022 282
- JP-A- 2017 042 379
- JP-U- 3 117 119

## Description

### Technical Field

The present invention relates to an absorbent article usable to absorb and retain excrements such as urine.

### Background Art

An absorbent article such as disposable diapers and sanitary napkins typically includes a topsheet to be placed relatively close to the skin of a wearer, a backsheet to be placed relatively distant from the skin of a wearer, and a liquid-retentive absorbent member between the sheets. As the absorbent member, a stack of a fiber material such as wood pulp or a member in which the stack supports absorbent polymer particles (hereinafter also collectively called a "stacked absorbent member") has been generally used. The stacked absorbent member is relatively bulky and thick and thus has excellent cushioning properties and the like, but gives an absorbent article having a poor or unattractive appearance due to its bulkiness. To address such disadvantages, as a thinner absorbent member than the stacked absorbent member, an absorbent member mainly including an absorbent polymer layer that is composed of substantially only an absorbent polymer and contains no fiber material (hereinafter also called a "sheet-type absorbent member") has been developed. As the sheet-type absorbent member, an absorbent member in which absorbent polymer particles are placed between two sheets placed to face together in the thickness direction is known.

Patent Literature 1 discloses an absorbent article including a central absorbent member having a two-layer structure of an upper absorbent member and a lower absorbent member and a pair of side absorbent members each including an extension outward in the lateral direction (width direction) from the corresponding side edge of the lower absorbent member along the longitudinal direction (length direction) of the upper absorbent member. An elastic member is provided on the side edge along the longitudinal direction of each side absorbent member, and the elastic member functions to allow the side absorbent member to rise toward the skin of a wearer wearing the absorbent article. Each of the upper absorbent member and the lower absorbent member includes a stacked absorbent member, and this makes the central absorbent member have a higher rigidity than the rigidity of the side absorbent members and allows the side absorbent members to satisfactory rise. In addition, the central absorbent member can absorb excrements sufficiently (Paragraph [0014] in Patent Literature 1).

Patent Literatures 2 and 3 disclose an absorbent article including an absorbent member having a two-layer structure. In the absorbent member, the upper layer to be placed relatively close to the skin of a wearer includes a stacked absorbent member, whereas the lower layer to be placed relatively distant from the skin of a wearer includes a sheet-type absorbent member. In Patent Literature 2, the upper layer and the lower layer have the same lateral dimension (width), whereas in Patent Literature 3, the lower layer is wider than the upper layer, and the sheet-type absorbent member of the lower layer has extensions extending outward in the lateral direction from the corresponding side edges along the longitudinal direction of the stacked absorbent member of the upper layer. Additional relevant prior art is represented by EP 2 901 992 A1, JP 3 117 119 U, JP 2016 022282 A, JP 2015 518773 A and JP 2017 042379 A.

### Citation List

### Patent Literatures

Patent Literature 1: JP 2008-237252 A
Patent Literature 2: US 2008/312621 A1
Patent Literature 3: US 2020/179187 A1

### Summary of Invention

The present invention relates to an absorbent article as defined in independent claim 1. Preferred aspects are defined in the dependent claims. Other features, effects, and embodiments of the present invention will be described below.

### Brief Description of Drawings

[FIG. 1] FIG. 1 is a developed plan view schematically showing a skin-facing surface side (topsheet side) of an open type disposable diaper in a spread out and stretched state as an embodiment of an absorbent article in the present invention.
[FIG. 2] FIG. 2 is a cross-sectional view schematically showing a cross section taken along line I-I in FIG. 1 (a cross section along the thickness direction of the crotch portion).
[FIG. 3] FIG. 3 is an enlarged cross-sectional view of the absorbent assembly in FIG. 2.
[FIG. 4] FIG. 4 is a plan view schematically showing a skin-facing surface side of a laminate structure of the absorbent member in the diaper in FIG. 1.
[FIGS. 5] FIG. 5(a) and FIG. 5(b) are cross-sectional views each schematically showing a laminate structure of the absorbent member in the crotch portion of the diaper in FIG. 1 at the time of wearing.
[FIG. 6] FIG. 6 is a view of an absorbent assembly in another embodiment of the absorbent article of the present invention, corresponding to FIG. 3 (a schematic cross-sectional view along the thickness direction in the crotch portion of the absorbent assembly).
[FIGS. 7] FIG. 7(a) and FIG. 7(b) are cross-sectional views each schematically showing a laminate structure of the absorbent member in the crotch portion of the absorbent article in FIG. 6 at the time of wearing.
[FIG. 8] FIG. 8 is a view of an absorbent assembly in still another embodiment of the absorbent article of the present invention, corresponding to FIG. 3.
[FIGS. 9] FIG. 9(a) is a view of an absorbent assembly in still another embodiment of the absorbent article of the present invention, corresponding to FIG. 3, and FIG. 9(b) is a cross-sectional view schematically showing a laminate structure of the absorbent member in the crotch portion of the absorbent article in FIG. 9(a) at the time of wearing.
[FIGS. 10] FIG. 10(a) is a view of an absorbent assembly in still another embodiment of the absorbent article of the present invention, corresponding to FIG. 3 (a schematic cross-sectional view along the thickness direction in the crotch portion of the absorbent assembly), and FIG. 10(b) is a cross-sectional view schematically showing a laminate structure of the absorbent member in the crotch portion of the absorbent article in FIG. 10(a) at the time of wearing.
[FIG. 11] FIG. 11 is a view of an open type disposable diaper as still another embodiment of the absorbent article of the present invention, corresponding to FIG. 1.
[FIG. 12] FIG. 12 is a view of a laminate structure of the absorbent member in the diaper in FIG. 11, corresponding to FIG. 4.

### Description of Embodiments

A conventional absorbent article has lower fitting properties to the crotch section of a wearer after liquid absorption. As a technique capable of solving the problem, a stacked absorbent member having a sandglass-like shape in plan view in which the central region in the longitudinal direction is narrowed has been developed. As another technique capable of solving the problem, a stacked absorbent member partially having portions without any forming material such as a fiber material, also called slit portions or the like, has been developed. In the absorbent members pertaining to these related arts, a fiber material included in the absorbent member is bonded together after liquid absorption, and thus the whole absorbent member is tightly packed and is accordingly difficult to deform when an external force such as a body pressure is applied. A portion of an absorbent member corresponding to the crotch section of a wearer wearing the absorbent article is sandwiched between the legs (thighs) of the wearer. When the portion is difficult to deform after liquid absorption, the portion may apply a higher pressure on the legs of the wearer after liquid absorption, and this may impair wearing comfort. Any technique capable of reducing the pressure of an absorbent member on the legs of a wearer after liquid absorption but capable of maintaining the liquid absorption capacity of the absorbent member has not been developed yet.

The present invention therefore relates to an absorbent article having excellent liquid absorbability, reducing the pressure on the legs of a wearer, and having excellent wearing comfort.

The present invention will now be described on the basis of preferred embodiments thereof with reference to drawings. In the following description of drawings, identical or similar components are indicated by an identical or similar sign. Drawings are basically schematic, and dimensional ratios and the like may differ from the actual ones.

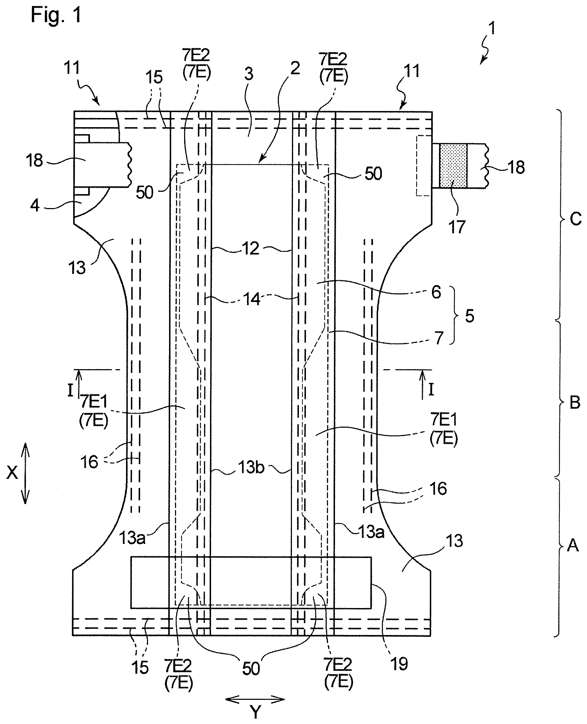

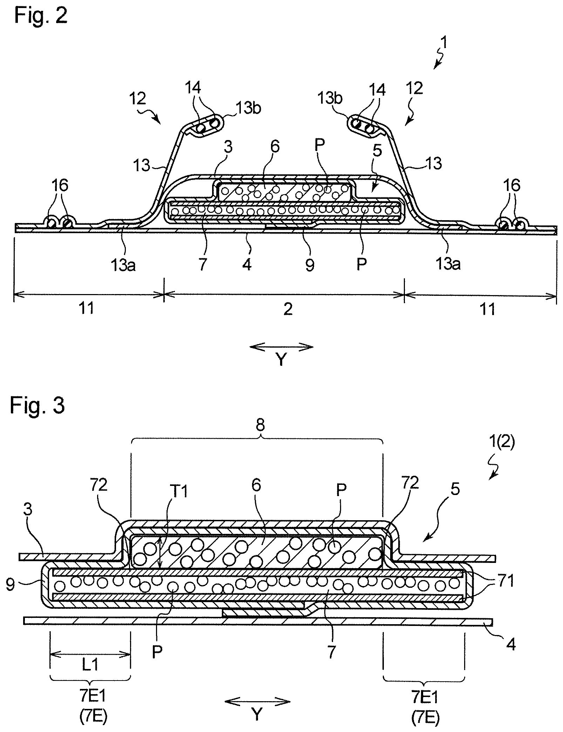

FIG. 1 and FIG. 2 show a disposable diaper 1 as an embodiment of the absorbent article of the present invention. The diaper 1 has a longitudinal direction X corresponding to the front-rear direction of a wearer, or to the direction extending from the front side through the crotch section to the rear side, and a lateral direction Y orthogonal to the longitudinal direction X. The diaper 1 is sectioned into three portions: a crotch portion B including an excretory-facing portion (not shown) to be placed on the crotch section of a wearer and to face the excretory including the penis; a front portion A to be placed closer to the front side (abdominal side) of a wearer than the crotch portion B; and a rear portion C to be placed closer to the rear side (dorsal side) of a wearer than the crotch portion B.

Each of the front portion A and the rear portion C typically includes a hip part to be placed on the below-waist of a wearer wearing the diaper 1. The front portion A is a part of the front body portion of the diaper 1, and the rear portion C is a part of the rear body portion of the diaper 1. The crotch portion B typically includes at least a part of the central portion in the longitudinal direction X of the diaper 1 and extends from the front body portion to the rear body portion of the diaper 1.

In the present invention, the front portion A, the crotch portion B, and the rear portion C may be trisected regions of a spread out and stretched diaper 1 in the longitudinal direction X. In the description, a "spread out and stretched state" is the state in which a diaper is spread out as shown in FIG. 1, and the spread out diaper is flattened out by stretching elastic members of each portion into design sizes (equal to the sizes of a flattened diaper where the effect of the elastic members is completely eliminated).

The diaper 1 includes an absorbent member 5 to absorb and retain a body fluid such as urine and a topsheet 3 placed on a skin-facing surface side of the absorbent member 5.

In the present embodiment, the diaper 1 includes an absorbent assembly 2, and the absorbent assembly 2 includes the topsheet 3, the absorbent member 5, and a backsheet 4 placed on a non-skin-facing surface side of the absorbent member 5. The absorbent assembly 2 extends in the longitudinal direction X from the front portion A to the rear portion C, and the length direction thereof is coincident with the longitudinal direction X. The topsheet 3 together with the leak-proof cuff forming sheets 13 described later forms a skin-facing surface (inner surface) of the diaper 1, and the backsheet 4 forms a non-skin-facing surface (outer surface) of the diaper 1. The constituent members of the absorbent assembly 2 are joined together by a known joining means such as an adhesive. As the constituent members of the absorbent assembly 2, any member that has been typically used in this kind of absorbent article may be used provided that the following description for the corresponding member is applicable if any. As the topsheet 3, various liquid-permeable sheets may be used, and examples include a nonwoven fabric, a woven fabric, and paper. As the backsheet 4, a leak-proof sheet, specifically, a liquid-impermeable sheet (through which no liquid passes) or a sparingly liquid-permeable sheet (through which liquid is not completely prevented from passing but is difficult to pass) may be used, and examples include a moisture permeable resin film and a laminate of the resin film and a nonwoven fabric.

In the present description, a "skin-facing surface" is one face of an absorbent article or a constituent member thereof (for example, an absorbent member) and is the face facing the skin of a wearer at the time of wearing the absorbent article or is the face relatively close to the skin of a wearer, and a "non-skin-facing surface" is the other face of an absorbent article or a constituent member thereof and is the face facing opposite to the skin of a wearer at the time of wearing the absorbent article or is the face relatively distant from the skin of a wearer. In the present description, "at the time of wearing" means a condition in which a typical, appropriate wearing position or a normal wearing position of the absorbent article is maintained.

The diaper 1 includes flaps 11 extending outward from the peripheral edges of the absorbent assembly 2, or from the peripheral edges of the absorbent member 5. The flaps 11 include members extending outward from the peripheral edges of the absorbent assembly 2. In the present embodiment, as shown in FIG. 2, the topsheet 3 overlies the whole region of the skin-facing surface of the absorbent member 5, and the backsheet 4 overlies the whole region of the non-skin-facing surface of the absorbent member 5. The sheets 3 and 4 further extend outward in the lateral direction Y from the respective side edges along the longitudinal direction X of the absorbent member 5 and form, together with the leak-proof cuff forming sheets 13 described later, side flaps that are each a part of the flap 11. The side flap is a portion in the flap 11 and is located outside in the lateral direction Y from each side edge along the longitudinal direction X of the absorbent member 5 and from an imaginary extension line of the side edge. A plurality of members included in the flap 11 are joined together by a known joining means such as an adhesive, heat sealing, and ultrasonic sealing.

As shown in FIG. 1 and FIG. 2, a pair of leak-proof cuffs 12 and 12 are provided along the respective sides along the longitudinal direction X of the absorbent assembly 2 and are to rise toward the skin of a wearer wearing the diaper. Each leak-proof cuff 12 includes a liquid resistant or water repellent and breathable leak-proof cuff forming sheet 13, and one side in the lateral direction Y of the sheet 13 is fixed to other members (in the embodiment in the figure, the topsheet 3 and the backsheet 4) to form a fixed end 13a, whereas the other side in the lateral direction Y is a free end 13b that is not fixed to other members. To the free end 13b of the sheet 13, leak-proof cuff forming elastic members 14 are fixed in a stretched state in the longitudinal direction X so as to be stretchable in the same direction. When the diaper 1 is worn, the shrinkage force of the elastic members 14 allows the free ends 13b of the sheets 13 at least in the crotch portion B to rise toward the wearer from the fixed ends 13a to other members as rising base ends, and accordingly, the pair of leak-proof cuffs 12 and 12 rise to prevent excrements such as urine from flowing outside in the lateral direction Y.

In the flaps 11 in the respective end portions (waist end portions) in the longitudinal direction X of the front portion A and the rear portion C, a plurality of waist gathering forming elastic members 15 are provided to be stretchable in the lateral direction Y, and these elastic members 15 are intermittently placed at certain intervals in the longitudinal direction X. The elastic members 15 are arranged while exhibiting the elasticity as described above, and thus in the waist end portions in the front portion A and the rear portion C where the elastic members are arranged, shrinkage of the elastic members 15 forms a plurality of gathers extending in a direction intersecting the shrinkage direction of the elastic members 15 on the sheets (in the present embodiment. the sheets 3 and 4) included in the waist end portions, in a natural state or worn state of the diaper 1. Accordingly, waist gathering in which the gathers substantially continue on the entire circumference of each waist end portion is formed.

In each flap 11, in a leg portion to be placed around the leg of a wearer wearing the diaper 1, leg gathering forming elastic members 16 extensible in the longitudinal direction X extend in the longitudinal direction X over the entire length in the longitudinal direction X at least in the crotch portion B. At the time of wearing the diaper 1, shrinkage of the elastic members 16 forms a plurality of gathers extending in a direction intersecting the shrinkage direction of the elastic members 16 on the sheets (in the present embodiment, the sheets 4 and 13) included in the leg portion. Accordingly, leg gathering in which the gathers substantially continue on the entire circumference of the leg of a wearer is formed.

These gathering forming elastic members 15 and 16 in a stretched state are interposed between a plurality of sheets (in the present embodiment, two sheets of the topsheet 3, the backsheet 4, and the leak-proof cuff forming sheet 13) included in the flap 11 and are fixed by a joining means such as an adhesive.

The above elastic members 14, 15, and 16 may be in any form, and filamentous members having, for example, a rectangular, regular square, circular, or polygonal cross section, string members (such as flat rubber), multifilament members, or the like may be used.

The diaper 1 is what is called an open type disposable diaper and includes, in each side edge portion along the longitudinal direction X in the rear portion C of the diaper 1, an attachment member 18 having an attachment portion 17 and, on the non-skin-facing surface of the backsheet 4 constituting the non-skin-facing surface of the front portion A, an attachment region 19 to which the attachment portions 17 are attachable, as shown in FIG. 1.

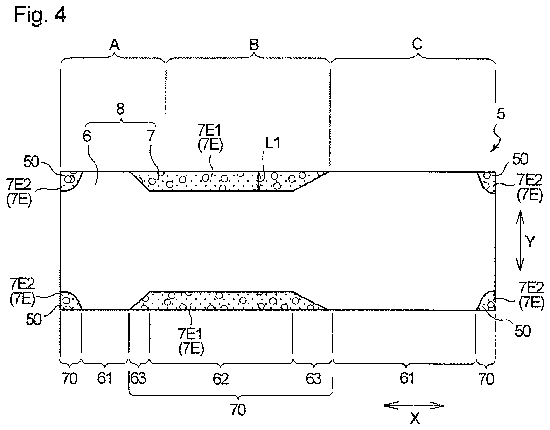

FIG. 3 and FIG. 4 show the absorbent member 5 in detail. The absorbent member 5 has a rectangular shape in such a plan view as shown in FIG. 4 and extends in the longitudinal direction X from the front portion A to the rear portion C, and the length direction thereof is coincident with the longitudinal direction X. The absorbent member 5 having a rectangular shape in plan view has two nook portions 50 in each of the front portion A and the rear portion C (four nook portions in total). The absorbent member 5 has a laminate structure 8 in which a first layer 6 and a second layer 7 are laminated in the thickness direction of the absorbent member 5 (in the vertical direction in FIG. 3). In the present embodiment, the laminate structure 8 is a two-layer structure of the first layer 6 and the second layer 7. The laminate structure 8 extends over the entire length in the longitudinal direction X of the absorbent member 5 and extends in the longitudinal direction X from the front portion A to the rear portion C, and the length direction thereof is coincident with the longitudinal direction X.

In the present embodiment, the absorbent member 5 includes a core-wrap sheet 9 overlying the outer surface of the laminate structure 8, and the skin-facing surface and the non-skin-facing surface of the laminate structure 8 are covered with the single core-wrap sheet 9. As the core-wrap sheet 9, a liquid permeable sheet may be used, and, for example, paper, a nonwoven fabric, or a woven fabric may be used. In the present invention, the absorbent member 5 may include no core-wrap sheet 9 or may include a plurality of core-wrap sheets 9. In the latter case, for example, the absorbent member 5 may include a single skin-facing core-wrap sheet overlying the skin-facing surface of the laminate structure 8 and a single non-skin-facing core-wrap sheet overlying the non-skin-facing surface of the laminate structure 8.

In the present embodiment, as shown in FIG. 3, the second layer 7 is placed on the non-skin-facing surface side of the first layer 6 and is placed more distant from the skin of a wearer wearing the diaper 1 than the first layer 6. Accordingly, in the present embodiment, a body fluid such as urine excreted by a wearer of the diaper 1 typically passes through the laminate structure 8 in the order of the first layer 6 and the second layer 7.

In the first layer 6, a stack of a fiber material (fiber aggregate) supports an absorbent polymer. Sign P in the drawings is an absorbent polymer. The first layer 6 is typically composed of a mixed layer of the fiber material and the absorbent polymer. The first layer 6 is what is called a stacked absorbent member.

In the first layer 6, the absorbent polymer may be uniformly distributed throughout the first layer 6 or may be unevenly distributed. The former is a typical distribution pattern. A specific example of the latter is a pattern in which an absorbent polymer is present mainly in the skin-facing surface side or the non-skin-facing surface side of the first layer 6 or a pattern in which the content of an absorbent polymer is larger in one of the skin-facing surface side and the non-skin-facing surface side than the other. In the description, the skin-facing surface side of a first layer 6 and the non-skin-facing surface side of the first layer 6 is defined by an imaginary straight line (not shown) that bisects the first layer 6 in the thickness direction.

As the fiber material included in the first layer 6, any material usable in this type of absorbent member may be used, and a single material or a mixture of two or more materials may be used. The fiber material is preferably an absorbent fiber material. Examples of the absorbent fiber material include natural fibers such as wood pulps including softwood pulp and hardwood pulp and non-wood pulps including cotton pulp and hemp pulp; modified pulps such as cationized pulp and mercerized pulp (i.e., cellulose fibers); and hydrophilic synthetic fibers.

As the absorbent polymer included in the first layer 6, any material usable in this type of absorbent member may be used, and typically a hydrogel material capable of absorbing and retaining water may be used. For example, a polymer or a copolymer of acrylic acid or an alkali metal acrylate may be used. Examples thereof include polyacrylic acid, salts thereof, polymethacrylic acid, and salts thereof and specifically include a partial sodium salt of acrylic polymer. The absorbent polymer may have any shape and may be, for example, spherical, tufted, massive, ellipsoid, fibrous, or indefinite particles or particles having a combination shape thereof.

The basis weight of the first layer 6, the content of a forming material, and the like are not specifically limited but are preferably set at the following values from the viewpoint of allowing the first layer 6 to certainly achieve intended effects.

The basis weight of the first layer 6 is preferably 10 g/m² or more and more preferably 50 g/m² or more and is preferably 600 g/m² or less and more preferably 500 g/m² or less.

The content of the fiber material in the first layer 6 is preferably 5% by mass or more and more preferably 10% by mass or more and is preferably 70% by mass or less and more preferably 65% by mass or less relative to the total mass of the first layer 6.

The content of the absorbent polymer in the first layer 6 is preferably 30% by mass or more and more preferably 35% by mass or more and is preferably 95% by mass or less and more preferably 90% by mass or less relative to the total mass of the first layer 6.

The first layer 6 may be produced by using a known fiber stacking apparatus with a rotating drum by a common method. The fiber stacking apparatus typically includes a rotating drum having, on the outer peripheral face, stacking recesses and a duct having a flow path therein for conveying forming materials (a fiber material and an absorbent polymer) to the stacking recesses. While the rotating drum is rotated about the rotating shaft in the drum circumferential direction, the forming materials conveyed on an air flow generated in the flow path by aspiration from the inside of the rotating drum are stacked on the stacking recesses. The stacked product formed in the stacking recess through such a stacking process is the first layer 6.

In the second layer 7, an absorbent polymer P is placed between two base sheets 71 and 71 placed to face together in the thickness direction of the second layer 7 (absorbent member 5), as shown in FIG. 3. The second layer 7 is what is called a sheet-type absorbent member.

An absorbent polymer placement layer (the layer between two base sheets 71 and 71) in the second layer 7 typically contains only an absorbent polymer as an absorbent material and contains no absorbent fiber material such as cellulose fibers or, if containing an absorbent fiber material, contains the absorbent fiber material at a smaller content (for example, 10% by mass or less, preferably 3% by mass or less, relative to the total mass of the absorbent polymer placement layer) than the content of the absorbent fiber material in the first layer 6. As the absorbent polymer included in the second layer 7, a similar absorbent polymer to the absorbent polymer included in the first layer 6 may be used.

In the second layer 7, in order to join two base sheets 71 and 71 or to join the base sheet 71 and the absorbent polymer, an adhesive such as a hot melt adhesive may be applied onto the surface (inner surface) that is of at least one of the sheets 71 and 71 and is to face the absorbent polymer placement layer.

As the base sheet 71 included in the second layer 7, a liquid permeable or liquid absorbable sheet may be used. The base sheet 71 is typically a fiber sheet mainly containing fibers or having a fiber content of more than 50% by mass.

Examples of the constituent fibers in the base sheet 71 include natural fibers such as wood pulps including softwood pulp and hardwood pulp and non-wood pulps including cotton pulp and hemp pulp; modified pulps such as cationized pulp and mercerized pulp (i.e., cellulose fibers); and synthetic fibers containing a resin such as polyethylene and polypropylene, and these materials may be used singly or in combination of two or more of them.

Example forms of the base sheet 71 include paper, woven fabric, and nonwoven fabric, and examples of the nonwoven fabric include air-through nonwoven fabric, heat roll nonwoven fabric, spunlace nonwoven fabric, spunbond nonwoven fabric, meltblown nonwoven fabric, and spunbond-meltblown-spunbond (SMS) nonwoven fabric. The base sheet 71 typically has a single-layer structure formed from one of these materials but may have a laminate structure in which two or more materials are laminated and integrated.

The basis weight of the base sheet 71 is not specifically limited but is preferably 5 g/m² or more and more preferably 8 g/m² or more and is preferably 50 g/m² or less and more preferably 30 g/m² or less from the viewpoint of allowing the second layer 7 to certainly achieve intended effects.

Two base sheets 71 and 71 included in the second layer 7 may be the same sheet or may differ in one or more characteristics including structure, material, and physical properties.

As shown in FIG. 1 to FIG. 4, the second layer 7 has, at least in the crotch portion B, crotch extensions 7E1 extending outward in the lateral direction Y from the respective side edges along the longitudinal direction X of the first layer 6 included in the laminate structure 8.

In the present embodiment, the second layer 7 has lateral extensions 7E extending outward in the lateral direction Y from the respective side edges along the longitudinal direction X of the first layer 6. The lateral extensions 7E include the crotch extensions 7E1 and nook extensions 7E2 that are located at nook portions 50 in the front portion A and the rear portion C of the absorbent member 5 and are not connected to the crotch extensions 7E1. More specifically, the diaper 1 is formed symmetrically about an imaginary center line (not shown) that extends in the longitudinal direction X and bisects the diaper 1 in the lateral direction Y into one side and the other side, and the absorbent member 5 has a rectangular shape in such a plan view as shown in FIG. 4. A pair of the crotch extensions 7E1 are formed at least in the crotch portion B while the imaginary center line is located therebetween, and a pair of the nook extensions 7E2 are formed at two nook portions 50 in each of the front portion A and the rear portion C of the absorbent member 5 while the imaginary center line is located therebetween.

In the present description, the explanation for the lateral extension 7E is applied to both the crotch extension 7E1 and the nook extension 7E2, and the explanation for one of the crotch extension 7E1 and the nook extension 7E2 is also applied to the other, unless otherwise specified.

In the present embodiment, as shown in FIG. 4, the ends in the longitudinal direction X of the first layer 6 are coincident with the ends in the longitudinal direction X of the second layer 7, and the second layer 7 does not extend outward in the longitudinal direction X from each end in the longitudinal direction X of the first layer 6 and from an imaginary extension line of each end in the longitudinal direction X (not having the longitudinal extension 7F described later). The second layer 7 has a rectangular shape in plan view and has a constant dimension (width) in the lateral direction Y over the entire length in the longitudinal direction X of the second layer 7, whereas the first layer 6 has varying widths and includes maximum width portions 61 having the maximum width and a minimum width portion 62 having the minimum width in the first layer 6. More specifically, the first layer 6 includes, in each of the front portion A and the rear portion C, a single maximum width portion 61 that is located a certain distance apart from the end in the longitudinal direction X of the first layer 6 inward in the longitudinal direction X, includes a single minimum width portion 62 between the maximum width portion 61 in the front portion A and the maximum width portion 61 in the rear portion C, and further includes, between one maximum width portion 61 and the minimum width portion 62 and between the other maximum width portion 61 and the minimum width portion 62, variable width portions 63 having widths that gradually increase from the inside to the outside in the longitudinal direction X. Each of the pair of crotch extensions 7E1 and 7E1 of the second layer 7 extends outward in the lateral direction Y from the minimum width portion 62 of the first layer 6 and from two variable width portions 63 and 63 continuing to the respective ends in the longitudinal direction X of the minimum width portion 62 and has a substantially trapezoidal shape in plan view. The crotch extensions 7E1 are located at least in the crotch portion B and may further extend to the front portion A and/or the rear portion C.

In the diaper 1, when the crotch portion B is compressed in the lateral direction Y between the legs (thighs) of a wearer wearing the diaper, the crotch extensions 7E1 affected by the compression are foldable along the side edges of the first layer 6 along the longitudinal direction X.

As described above, the diaper 1 in the embodiment has, as the lateral extensions 7E, the crotch extensions 7E1 located in the crotch portion B and the nook extensions 7E2 located at the end portions (nook portions 50) in the longitudinal direction X in the front portion A and the rear portion C, and the extensions 7E1 and 7E2 are foldable when compressed in the lateral direction Y, but the crotch extensions 7E1 can be certainly folded when the crotch portion B is compressed in the lateral direction Y between the legs of a wearer. The nook extensions 7E2 are located apart from the crotch portion B and are not connected to the crotch extensions 7E1 (the first layer 6 (maximum width portions 61) is located between the extensions 7E1 and 7E2). Hence, when the crotch portion B is compressed in the lateral direction Y between the legs of a wearer, the nook extensions may not be folded depending on compression effect or may be folded to a smaller degree than the crotch extensions 7E1 if folded. However, even when the nook extensions 7E2 are not folded at the time of wearing the diaper 1, simply providing the nook extensions 7E2 effectively improves the cushioning properties of the absorbent member 5 to enhance the wearing comfort of the diaper 1 provided that the magnitude relations (specifically the second magnitude relation) described later to the first layer 6 in terms of an absorbent polymer is satisfied.

When the lateral extensions 7E (the crotch extensions 7E1, and the nook extensions 7E2) are compressed in the lateral direction Y, the boundaries between the lateral extensions 7E and the first layer 6 (the laminate structure 8) (the overlapping portions in plan view in the second layer 7 with the side edges along the longitudinal direction X of the first layer 6) function as buckling guide portions 72, and the lateral extensions 7E are folded in the lateral direction Y

The buckling guide portion 72 is naturally formed typically by a difference in rigidity between the lateral extension 7E and the first layer 6 (laminate structure 8). In other words, in a condition in which typically the lateral extensions 7E, more typically the crotch extensions 7E1 have a lower rigidity than the first layer 6 (laminate structure 8), "when the crotch portion B is compressed in the lateral direction Y between the legs of a wearer wearing the diaper 1, the lateral extensions 7E (crotch extensions 7E1) affected by the compression are foldable along the side edges of the first layer 6 along the longitudinal direction X". Specific examples of the embodiment in which the lateral extension 7E has a lower rigidity than the first layer 6 (laminate structure 8) include an embodiment in which the former has a lower density than the latter and an embodiment in which the former is thinner than the latter.

As another method of forming the buckling guide portion 72 except a rigidity difference between the lateral extensions 7E and the first layer 6 (laminate structure 8), for example, a linear buckling guide portion 72 such as a folding line and perforations may be formed at an intended forming position of the buckling guide portion 72 in the second layer 7 (for example, at the overlapping portions in plan view in the second layer 7 with the side edges along the longitudinal direction X of the first layer 6).

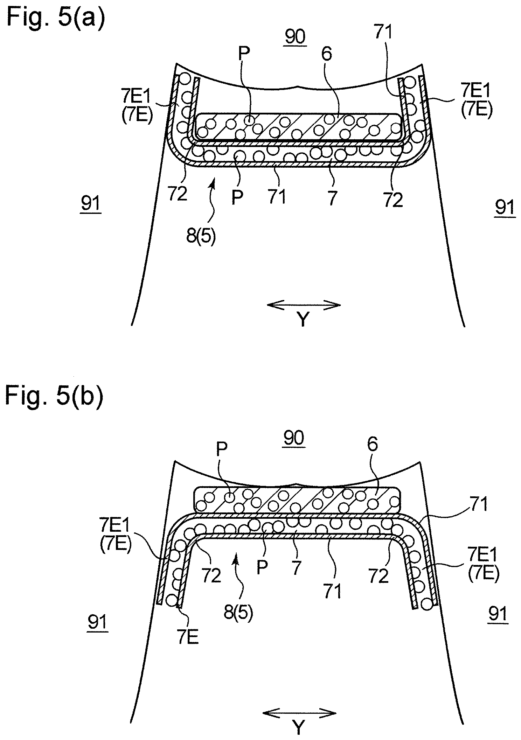

The folding direction of the crotch extensions 7E1 when compressed in the lateral direction Y is toward the skin of a wearer wearing the diaper 1 or is opposite to the skin (non-skin-facing side). With reference to FIGS. 5, when the crotch portion B is compressed in the lateral direction Y between the legs 91 and 91 of a wearer wearing the diaper 1, the crotch extensions 7E1 are folded to rise toward the crotch section 90 of the wearer (see FIG. 5(a)) or are folded to rise opposite to the crotch section 90 (see FIG. 5(b)). In each embodiment of FIG. 5(a) and FIG. 5(b), the rising crotch extensions 7E1 come into facial contact with the insides of the legs 91 of a wearer and function as a cushion, and thus the pressure on the leg 91 from a tightly packed absorbent member 5 after liquid absorption can be reduced. In particular, in the embodiment of FIG. 5(a), the side edge portions along the longitudinal direction X of the first layer 6, which can mainly cause a pressure on the legs 91 of a wearer, are covered with the crotch extensions 7E1 rising toward the crotch section 90 and are prevented from coming into contact with the legs 91. Hence, even when the side edge portions are tightly packed after liquid absorption, the cushioning effect by the crotch extensions 7E1 rising between the side edge portions and the legs 91 can effectively reduce the pressure on the legs 91. In terms of the effect of reducing the pressure on the legs of a wearer, the embodiment of FIG. 5(a) is superior to the embodiment of FIG. 5(b).

The folding direction when the lateral extension 7E is compressed in the lateral direction Y may be controlled, for example, by forming a linear buckling guide portion 72 such as a folding line and perforations at an intended forming position of the buckling guide portion 72. Specifically, for example, to allow the crotch extension 7E1 to rise toward the crotch section 90 as in the embodiment of FIG. 5(a), a folding line may be previously formed at an intended forming position of an intended buckling guide portion 72 (for example, the overlapping portion in plan view in the second layer 7 with the side edge along the longitudinal direction X of the first layer 6) by folding the crotch extension 7E1 at the intended forming position toward the skin-facing surface side of the second layer 7. To allow the crotch extension 7E1 to rise opposite to the crotch section 90 as in the embodiment of FIG. 5(b), the same process as in the embodiment of FIG. 5(a) may be performed except that the crotch extension 7E1 is folded toward the non-skin-facing surface side of the second layer 7.

The diaper 1 is characterized in that, in addition to the crotch extensions 7E1 of the second layer 7 that are foldable along the side edges of the first layer 6 along the longitudinal direction X as described above, the first layer 6 has a larger absorbent polymer basis weight (total mass of absorbent polymers per unit area of the layer) than the second layer 7, and the second layer 7 has a larger absorbent polymer density (total mass of absorbent polymers per unit volume of the layer) than the first layer 6, in a region coincident with the crotch extensions 7E1 in the longitudinal direction X.

In the present embodiment, the lateral extensions 7E (the crotch extensions 7E1, and the nook extensions 7E2) are foldable along the side edges of the first layer 6 along the longitudinal direction X, as well as in regions coincident with the lateral extensions 7E in the longitudinal direction X (hereinafter also called "lateral extension existence regions 70"), the above magnitude relations, or the magnitude relation "the first layer 6 > the second layer 7" in terms of absorbent polymer basis weight (hereinafter also called a "first magnitude relation") and the magnitude relation "first layer 6 < the second layer 7" in terms of absorbent polymer density (hereinafter also called a "second magnitude relation") are each satisfied.

In the present invention, the magnitude relations between the first layer 6 and the second layer 7 in terms of absorbent polymer basis weight and absorbent polymer density may be satisfied at least in the crotch portion B, are preferably satisfied also in the front portion A and/or the rear portion C, and are more preferably satisfied in all the lateral extension existence regions 70.

In the present embodiment, as shown in FIG. 4, the lateral extension existence regions 70 are three regions of 1) a region coincident with the crotch extensions 7E1 in the longitudinal direction X , 2) a region coincident with the nook extensions 7E2 in the front portion A in the longitudinal direction X, and 3) a region coincident with the nook extensions 7E2 in the rear portion C in the longitudinal direction X, and in each of the three regions, the first and second magnitude relations are satisfied.

In the present embodiment, an absorbent polymer is uniformly distributed in the whole second layer 7, and thus the second layer 7 has a constant absorbent polymer basis weight and a constant absorbent polymer density, but the present invention encompasses an embodiment in which the second layer 7 partly has varying absorbent polymer basis weights or varying absorbent polymer densities.

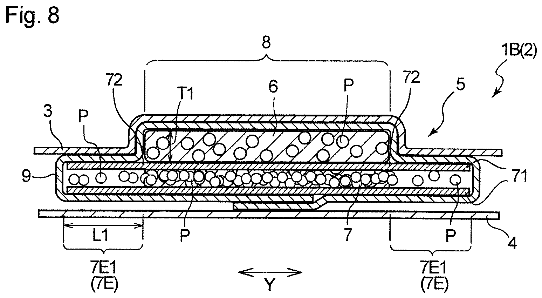

Regarding the first magnitude relation, in the case in which the second layer 7 partly has varying absorbent polymer basis weights, the first magnitude relation is satisfied when the magnitude relation "the absorbent polymer basis weight of the first layer 6 > the maximum absorbent polymer basis weight of the second layer 7" is satisfied. For example, as in the diaper 1B (see FIG. 8) described later, when the second layer 7 in a portion included in the laminate structure 8 (an overlapping portion with the first layer 6 in plan view) has a larger absorbent polymer basis weight than lateral extensions 7E located outward in the lateral direction Y from the above portion, at least the portion may have a smaller absorbent polymer basis weight than the absorbent polymer basis weight of the first layer 6.

Regarding the second magnitude relation, in the case in which the second layer 7 partly has varying absorbent polymer densities, the second magnitude relation is satisfied when the magnitude relation "the absorbent polymer density of the first layer 6 < the minimum absorbent polymer density of the second layer 7" is satisfied.

The first layer 6 has a main role of ensuring the absorption capacity of the absorbent member 5. When the first magnitude relation is satisfied, and accordingly the first layer 6 has a larger absorbent polymer basis weight than the second layer 7, the first layer 6 has a larger absorption capacity, and consequently, the diaper 1 can have a higher liquid absorbability.

When the second magnitude relation is satisfied, and accordingly the second layer 7 has a larger absorbent polymer density than the first layer 6, the second layer 7 easily swells due to liquid absorption to have excellent cushioning properties after liquid absorption, and the lateral extensions 7E, which are parts of the second layer 7, improve the above cushioning effect to result in an improvement in wearing comfort of the diaper 1. Specifically the crotch extensions 7E1 of the lateral extensions 7E are located in the crotch portion B and thus greatly contribute to the effect of reducing the pressure on the legs of a wearer. When the second magnitude relation is satisfied, and accordingly the crotch extensions 7E1 have a larger absorbent polymer density than the absorbent polymer density of the first layer 6 in a portion coincident with the crotch extensions 7E1 in the longitudinal direction X, the pressure on the legs of a wearer can be effectively reduced.

As described above, a diaper 1 satisfying the first and second magnitude relations has excellent liquid absorbability and wearing comfort.

In addition, in the present embodiment, the absorbent member 5 has the nook extensions 7E2 at the nook portions 50 in each of the front portion A and the rear portion C and thus has higher cushioning properties at each nook portion 50, and this further improves the wearing comfort of the diaper 1.

In the lateral extension existence region 70, the ratio of the absorbent polymer basis weight of the first layer 6 to the absorbent polymer basis weight of the second layer 7, in terms of the former/the latter, is preferably 1.1 or more and more preferably 1.2 or more and is preferably 3.0 or less and more preferably 2.5 or less provided that the former > the latter.

In the lateral extension existence region 70, the absorbent polymer basis weight of the first layer 6 is preferably 55 g/m² or more and more preferably 70 g/m² or more and is preferably 450 g/m² or less and more preferably 300 g/m² or less provided that the absorbent polymer basis weight of the first layer 6 is larger than the absorbent polymer basis weight of the second layer 7.

In the lateral extension existence region 70, the absorbent polymer basis weight of the second layer 7 is preferably 50 g/m² or more and more preferably 60 g/m² or more and is preferably 150 g/m² or less and more preferably 120 g/m² or less provided that the absorbent polymer basis weight of the second layer 7 is smaller than the absorbent polymer basis weight of the first layer 6.

In the lateral extension existence region 70, the ratio of the absorbent polymer density of the first layer 6 to the absorbent polymer density of the second layer 7 (particularly the lateral extension 7E), in terms of the former/the latter, is preferably 0.05 or more and more preferably 0.07 or more and is preferably 0.5 or less and more preferably 0.4 or less provided that the former < the latter.

In the lateral extension existence region 70, the absorbent polymer density of the first layer 6 is preferably 0.05 g/cm³ or more and more preferably 0.07 g/cm³ or more and is preferably 1.0 g/cm³ or less and more preferably 0.8 g/cm³ or less provided that the absorbent polymer density of the first layer 6 is smaller than the absorbent polymer density of the second layer 7 (particularly the lateral extension 7E).

In the lateral extension existence region 70, the absorbent polymer density of the second layer 7 (particularly the lateral extension 7E) is preferably 1.0 g/cm³ or more and more preferably 1.2 g/cm³ or more and is preferably 2.0 g/cm³ or less and more preferably 1.8 g/cm³ or less provided that the absorbent polymer density of the second layer 7 (particularly the lateral extension 7E) is larger than the absorbent polymer density of the first layer 6.

The absorbent polymer density of each layer (the first layer 6, and the second layer 7) included in the laminate structure 8 of an absorbent member 5 may be determined by the following method.

### <Measurement method of absorbent polymer density>

For the above diaper 1 as an example, first, five absorbent assemblies 2 as the measurement object are prepared. From each absorbent assembly 2, members other than the absorbent member 5, such as the topsheet 3, are removed, and the prepared absorbent member 5 (the laminate structure 8) is placed on a horizontal place without forming any creases or folding. From each layer (the first layer 6, the second layer 7) to be measured in the absorbent member 5, a regular square region in plan view having a dimension of 70 mm in the longitudinal direction X and a dimension of 70 mm in the lateral direction Y is cut out as a measurement sample. The thickness of the measurement sample is determined under a load of 0.6 g/cm². The thickness of the measurement sample may be determined, for example, by using a laser displacement meter (LK-080) manufactured by Keyence Corporation. To determine the thickness of a measurement sample, a regular square plate adjusted to give a load of 0.6 g/cm² (a 50 × 50 mm acrylic plate having a thickness of about 5 mm) is placed on the measurement sample, and the thickness of the measurement sample is determined by using the above laser displacement meter. The thickness of the measurement sample is determined at any five points in the surface direction by the above method, and the average is calculated as the thickness of the measurement sample (the first layer 6, or the second layer 7). The absorbent polymer basis weight of each of the layers 6 and 7 is divided by the thickness of the corresponding layer 6 or 7 to give an intended absorbent polymer density of the first layer 6 or the second layer 7.

With reference to FIG. 3 and FIG. 4, the lateral dimension L1 of the crotch extension 7E1 is preferably larger than the thickness T1 of the first layer 6 in a portion coincident with the crotch extension 7E1 in the longitudinal direction X. The lateral dimension L1 is the dimension in the lateral direction Y of the crotch extension 7E1 at a portion having the largest extension dimension in the lateral direction Y from the first layer 6 and, in the present embodiment, is the dimension in the lateral direction Y of the crotch extension 7E1 corresponding to the minimum width portion 62 of the first layer 6 in the crotch portion B. When the crotch extensions 7E1 are, for example, sandwiched between the legs of a wearer wearing the diaper 1 and are folded to rise as shown in FIGS. 5, satisfying the magnitude relation "the lateral dimension L1 of the crotch extension 7E1 > the thickness T1 of the first layer 6" increases the contact area between the rising crotch extensions 7E1 and the legs of a wearer, and thus the cushioning effect by the crotch extensions 7E1 can be further improved. Particularly in the embodiment of FIG. 5(a) or in the embodiment in which the crotch extensions 7E1 are folded to rise toward the crotch section 90 of a wearer, when the magnitude relation "the lateral dimension L1 of the crotch extension 7E1 > the thickness T1 of the first layer 6" is satisfied in the crotch extension 7E1 and in the first layer 6 in a portion coincident with the crotch extension 7E1 in the longitudinal direction X, the side edge portions along the longitudinal direction X of the first layer 6 are likely to be covered with the crotch extensions 7E1 rising toward the crotch section 90, and thus such a condition is effective.

The region where the magnitude relation "the lateral dimension L1 of the crotch extension 7E1 > the thickness T1 of the first layer 6" is satisfied may be present at least in the crotch portion B, is preferably present in 50% or more of the entire length in the longitudinal direction X of the crotch portion B in the crotch portion B, and more preferably extends over the entire length in the longitudinal direction X of the crotch portion B.

The lateral dimension of the nook extension 7E2 is preferably larger than the thickness T1 of the first layer 6 in a portion coincident with the nook extension 7E2 in the longitudinal direction X. In other words, the magnitude relation "the lateral dimension of the nook extension 7E2 > the thickness T1 of the first layer 6" is preferably satisfied.

The ratio of the lateral dimension of the lateral extension 7E (the lateral dimension L1 for the crotch extension 7E1) to the thickness T1 of the first layer 6, in terms of the former/the latter, is preferably 1.1 or more and more preferably 1.5 or more and is preferably 25.0 or less and more preferably 15.0 or less provided that the former > the latter.

The lateral dimension of the lateral extension 7E (the lateral dimension L1 for the crotch extension 7E1) is preferably 7.0 mm or more and more preferably 10.0 mm or more and is preferably 35.0 mm or less and more preferably 30.0 mm or less provided that the lateral dimension of the lateral extension 7E (the lateral dimension L1 for the crotch extension 7E1) is larger than the thickness T1 of the first layer 6.

The thickness T1 of the first layer 6 is preferably 1.5 mm or more and more preferably 2.0 mm or more and is preferably 6.0 mm or less and more preferably 5.5 mm or less provided that the thickness T1 of the first layer 6 is smaller than the lateral dimension of the lateral extension 7E (the lateral dimension L1 for the crotch extension 7E1).

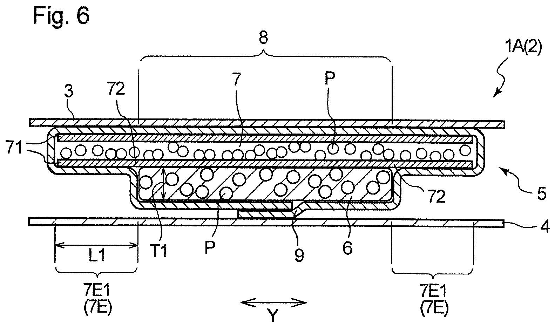

FIG. 6 to FIG. 12 show other embodiments of the absorbent article of the present invention. In the other embodiments described below, components different from those in the diaper 1 described above will be mainly described, and a similar component is indicated by an identical sign and is not described. To components not specifically described in the other embodiments described below, the description for the above diaper 1 is appropriately applied.

In a diaper 1A shown in FIG. 6, the second layer 7 is placed on the skin-facing surface side of the first layer 6 and is placed to be closer to the skin of a wearer wearing the diaper 1A than the first layer 6. Accordingly, in the diaper 1A, a body fluid such as urine excreted by a wearer typically passes through the laminate structure 8 in the order of the second layer 7 and the first layer 6.

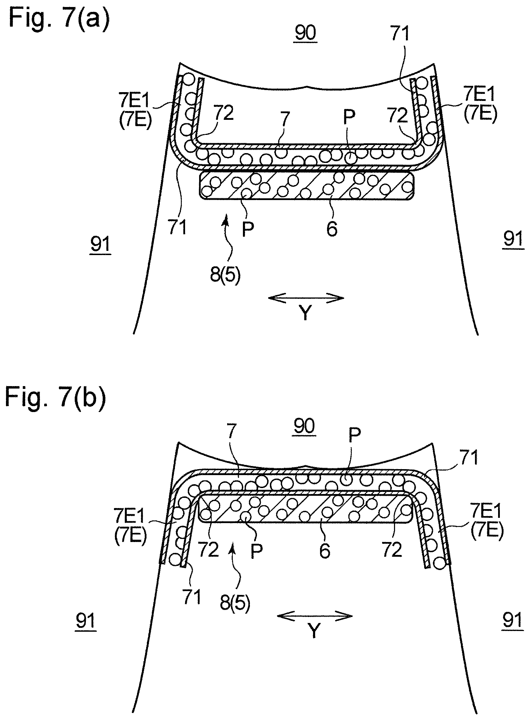

When the crotch portion B is compressed in the lateral direction Y between the legs 91 and 91 of a wearer wearing the diaper 1A, the crotch extensions 7E1 are folded to rise toward the crotch section 90 of the wearer as shown in FIG. 7(a) or are folded to rise opposite to the crotch section 90 as shown in FIG. 7(b). In each embodiment of FIG. 7(a) and FIG. 7(b), the rising crotch extensions 7E1 come into facial contact with the insides of the legs 91 of a wearer and function as a cushion, and thus the pressure on the legs 91 from a tightly packed absorbent member 5 after liquid absorption can be reduced. In particular, in the embodiment of FIG. 7(b), the side edge portions along the longitudinal direction X of the first layer 6, which can mainly cause a pressure on the legs 91 of a wearer, are covered with the crotch extensions 7E1 rising opposite to the crotch section 90 and are prevented from coming into contact with the legs 91. Hence, even when the side edge portions are tightly packed after liquid absorption, the cushioning effect by the crotch extensions 7E1 rising between the side edge portions and the legs 91 can effectively reduce the pressure on the legs 91. In terms of the effect of reducing the pressure on the legs of a wearer, the embodiment of FIG. 7(b) is superior to the embodiment of FIG. 7(a).

Of the four embodiments shown in FIGS. 5 and FIGS. 7, particularly preferred is the embodiment of FIG. 5(a), or the embodiment in which the second layer 7 is placed on the non-skin-facing surface side of the first layer 6, and when the absorbent article is compressed in the lateral direction Y at the time of wearing, the lateral extensions 7E (at least the crotch extensions 7E1) affected by the compression are foldable along the side edges of the first layer 6 along the longitudinal direction X to rise toward the crotch section 90 of the wearer.

In a diaper 1B shown in FIG. 8, the second layer 7 in a portion included in the laminate structure 8 (the second layer 7 in a portion overlapping with the first layer 6 in plan view) has a larger basis weight of the absorbent polymer P than the crotch extensions 7E1 in the lateral extension existence region 70. Satisfying such a magnitude relation improves the absorption capacity of the whole laminate structure 8, and the diaper 1B should have a higher liquid absorbability. In the present embodiment, not only the crotch extensions 7E1 but also the nook extensions 7E2 satisfy the above magnitude relation, or the second layer 7 in a portion included in the laminate structure 8 has a larger basis weight of the absorbent polymer P than the lateral extensions 7E (the crotch extensions 7E1, the nook extensions 7E2).

The ratio of the absorbent polymer basis weight of the second layer 7 in a portion included in the laminate structure 8 to the absorbent polymer basis weight of the lateral extension 7E, in terms of the former/the latter, is preferably 1.1 or more and more preferably 1.2 or more and is preferably 3.0 or less and more preferably 2.5 or less provided that the former > the latter.

The absorbent polymer basis weight of the second layer 7 in a portion included in the laminate structure 8 is preferably 55 g/m² or more and more preferably 70 g/m² or more and is preferably 450 g/m² or less and more preferably 300 g/m² or less provided that the absorbent polymer basis weight of the second layer 7 in a portion included in the laminate structure 8 is larger than the absorbent polymer basis weight of the lateral extension 7E.

The absorbent polymer basis weight of the lateral extension 7E is preferably 50 g/m² or more and more preferably 60 g/m² or more and is preferably 120 g/m² or less and more preferably 150 g/m² or less provided that the absorbent polymer basis weight of the lateral extension 7E is smaller than the absorbent polymer basis weight of the second layer 7 in a portion included in the laminate structure 8.

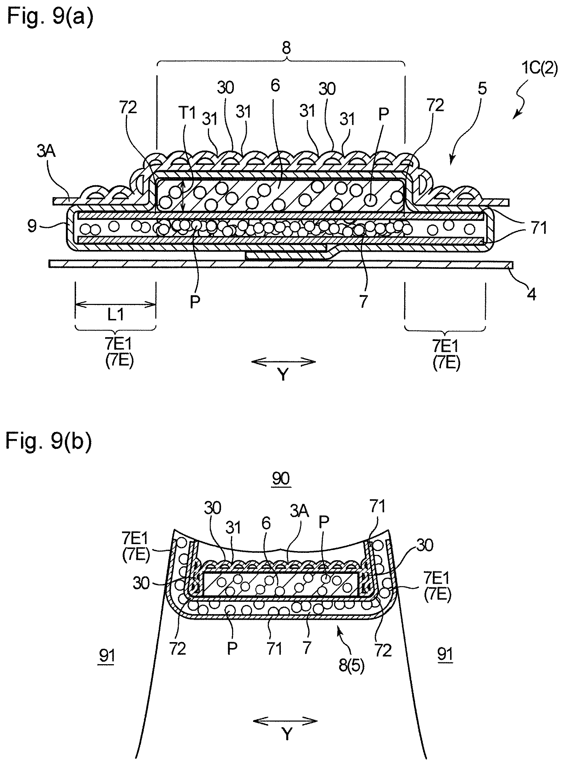

In a diaper 1C shown in FIGS. 9, a topsheet 3A extends in the lateral direction Y from the laminate structure 8 to the lateral extensions 7E (the crotch extensions 7E1, the nook extensions 7E2), and on the skin-facing surface of the topsheet 3A, a projecting-and-depressed region including a plurality of projections 30 projecting toward the skin of a wearer and depressions 31 located around the projections 30 is formed (see FIG. 9(a)).

The projecting-and-depressed region of the topsheet 3A is present at least in the crotch portion B and may also be present in the front portion A and/or the rear portion C. In the diaper 1C, the projecting-and-depressed region extends in the longitudinal direction X over the entire length in the longitudinal direction X of the diaper 1C. In the diaper 1C, the plurality of projections 30 have the same shape and the same size.

When the lateral extensions 7E, specifically the crotch extensions 7E1 are folded to rise toward the crotch section 90 of a wearer wearing the diaper 1C, the projections 30 of the topsheet 3A are present between the rising crotch extensions 7E1 and the side edge portions along the longitudinal direction X of the first layer 6 (see FIG. 9(b)). The projections 30 of the topsheet 3A between the rising crotch extension 7E1 and the first layer 6 function as a cushion as with the crotch extension 7E1, and thus the diaper 1C has an excellent effect of reducing the pressure on the legs 91 of a wearer thanks to the cushioning effect of both the crotch extensions 7E1 and the projections 30.

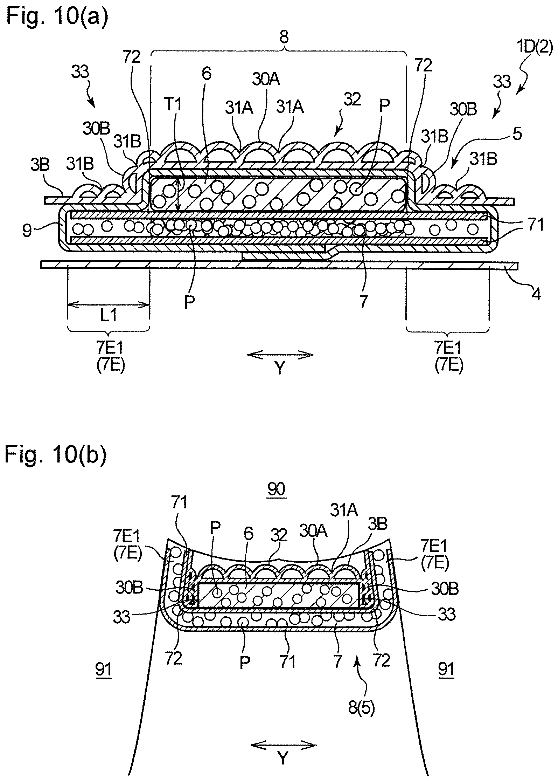

In a diaper 1D shown in FIGS. 10, a topsheet 3B extends in the lateral direction Y from the laminate structure 8 to the lateral extensions 7E, and on the skin-facing surface of the topsheet 3B, a projecting-and-depressed region including a plurality of projections 30A and 30B projecting toward the skin of a wearer and depressions 31A and 31B located around the projections 30A and 30B is formed. The projecting-and-depressed region of the topsheet 3B has a first region 32 that overlaps with the laminate structure 8 in plan view and second regions 33 that overlap with the lateral extensions 7E, specifically with the crotch extensions 7E1 in plan view, and the projections 30A in the first region 32 have a larger projecting height or a larger area than the projections 30B in the second region 33 (see FIG. 10(a)).

The projecting-and-depressed region of the topsheet 3B is present at least in the crotch portion B and may also be present in the front portion A and/or the rear portion C. In the diaper 1D, the projecting-and-depressed region extends in the longitudinal direction X over the entire length in the longitudinal direction X of the diaper 1D.

When the lateral extensions 7E, specifically the crotch extensions 7E1 are folded to rise toward the crotch section 90 of a wearer wearing the diaper 1D, the projections 30B in the second regions 33 of the topsheet 3B are present between the rising crotch extensions 7E1 and the side edge portions along the longitudinal direction X of the first layer 6 (see FIG. 10(b)).

The diaper 1D exerts substantially the same effect as that of the diaper 1C. Particularly in the diaper 1D, the projections 30A in the first region 32 capable of coming into contact with the crotch section 90 of a wearer wearing the diaper are large projections having a relatively large projecting height or a relatively large area, and this further improves touch feeling to the crotch section 90. In addition, the projections 30B in the second regions 33 to be present between the rising crotch extensions 7E1 and the first layer 6 and functioning as a cushion are small projections having a relatively small projecting height or a relatively small area, and this achieves an excellent cushioning effect while suppressing the thickness between the crotch extension 7E1 and the first layer 6. Accordingly, the liquid absorbability and the wearing comfort should be further improved.

The projections 30A in the first region 32 (large projections) may differ from the projections 30B in the second regions 33 (small projections) in either projecting height or area or in both projecting height and area.

The above "projecting height of the projections 30A in the first region 32" means the average of the projecting heights of the plurality of projections 30A in the first region 32, and the above "area of the projections 30A in the first region 32" means the average of the areas of the plurality of projections 30A in the first region 32. The same is applied to the projecting height and the area of the projections 30B in the second region 33.

From the viewpoint of more certainly achieving the above advantageous effect, the dimension (width) in the lateral direction Y of the first region 32 in the topsheet 3B is preferably 15% or more and more preferably 20% or more and is preferably 50% or less and more preferably 45% or less relative to the width (minimum width) of the diaper 1D.

From a similar viewpoint, the dimension (width) in the lateral direction Y of the second region 33 in the topsheet 3B is preferably 10% or more and more preferably 15% or more and is preferably 40% or less and more preferably 35% or less relative to the width (minimum width) of the diaper 1D.

The ratio of the projecting height of the projection 30A (large projection) to the projecting height of the projection 30B (small projection), in terms of the former/the latter, is preferably 1.2 or more and more preferably 1.5 or more and is preferably 10.0 or less and more preferably 8.0 or less provided that the former > the latter.

The projecting height of the projection 30A (large projection) is preferably 0.5 mm or more and more preferably 1.0 mm or more and is preferably 5.0 mm or less and more preferably 4.0 mm or less provided that the projecting height of the projection 30A (large projection) is larger than the projecting height of the projection 30B (small projection).

The projecting height of the projection 30B (small projection) is preferably 0.3 mm or more and more preferably 0.6 mm or more and is preferably 3.0 mm or less and more preferably 2.5 mm or less provided that the projecting height of the projection 30B (small projection) is smaller than the projecting height of the projection 30A (large projection).

The ratio of the area of the projection 30A (large projection) to the area of the projection 30B (small projection), in terms of the former/the latter, is preferably 2.0 or more and more preferably 3.0 or more and is preferably 10.0 or less and more preferably 8.0 or less provided that the former > the latter

The area of the projection 30A (large projection) is preferably 0.8 mm² or more and more preferably 3.0 mm² or more and is preferably 100.0 mm² or less and more preferably 80.0 mm² or less provided that the area of the projection 30A (large projection) is larger than the area of the projection 30B (small projection).

The area of the projection 30B (small projection) is preferably 0.2 mm² or more and more preferably 0.5 mm² or more and is preferably 50.0 mm² or less and more preferably 20.0 mm² or less provided that the area of the projection 30B (small projection) is smaller than the area of the projection 30A (large projection).

The number of projections 30A (large projections) in a unit area (1,000 mm²) in the first region 32 is preferably 50/1,000 mm² or more and more preferably 100/1,000 mm² or more and is preferably 1,000/1,000 mm² or less and more preferably 500/1,000 mm² or less.

The number of projections 30B (small projections) in a unit area (1,000 mm²) in the second region 33 is preferably 65/1,000 mm² or more and more preferably 200/1,000 mm² or more and is preferably 2,000/1,000 mm² or less and more preferably 1,000/1,000 mm² or less.

The projections 30 on the topsheet 3A included in the diaper 1C (see FIGS. 9) may be designed as with the projecting height, the area, and the number in a unit area of the projections 30A or the projections 30B.

The projecting height, the area, and the number of projections in a unit area on a topsheet may be determined by the following methods.

### <Measurement method of projecting height of projection on topsheet>

A projecting-and-depressed region of a topsheet to be measured is cut in the thickness direction by using a cutting tool such as a sharp razor. The section is observed to measure the distance between a position (a depression) adjacent to a projection and the top of the projection on the skin-facing surface of the projecting-and-depressed region, or to measure the shortest distance between an imaginary line that passes through the bottom of a depression and extends in the horizontal direction and an imaginary line that passes through the top of a projection and extends in the horizontal direction (the length of a normal orthogonal to the imaginary lines, between the imaginary lines), under no load, to give the projecting height of the projection. The projecting height of a projection corresponds to the apparent thickness of a topsheet. When a section is difficult to observe with the naked eye, the section may be observed under, for example, a microscope (VHX-1000 manufactured by KEYENCE) at a magnification of 20 to 100× for measurement.

In each of five regions arbitrarily selected from a single target region (for example, a first region 32 or a second region 33), the projecting height of a projection is determined by the above procedure, and the average of the measured values in these five regions is calculated as the projecting height of the projection in the target region.

### <Measurement method of area of projection on topsheet>

From a topsheet to be measured, a quadrangular shape in plan view having a longitudinal length of 50 mm and a lateral direction length of 20 mm is cut out as a test piece. The skin-facing surface of the test piece under no load is observed under a microscope (for example, a microscope manufactured by KEYENCE, VHX-1000) at an observation magnification of 10 to 100×. Next, the centroids of a plurality of depressions formed to surround a single projection are connected with straight lines. An area of the region surrounded by the straight lines and having a polygonal shape in plan view is regarded as the area of the projection, and the area of the projection is determined by using an image analysis software. When a topsheet has multiple types of projections different in projecting height or area, the area of each type of projection (for example, for the topsheet 3B of the above diaper 1D, each of the projections 30A and 30B) is determined by substantially the same procedure as above. In each of three regions arbitrarily selected from a single target region (for example, a first region 32 or a second region 33), the area of a projection is determined by the above procedure, and the average of the measured values in these three regions is calculated as the area of the projection in the target region.

### <Measurement method of number of projections in unit area of topsheet>

From a topsheet to be measured, a quadrangular shape in plan view having a longitudinal length of 50 mm and a lateral direction length of 20 mm is cut out as a test piece. The test piece is observed under a microscope (for example, a microscope manufactured by KEYENCE, VHX-1000) at an observation magnification of 20 to 100×, and the number of projections of the test piece is determined. The determined value is divided by the area (1,000 mm²) of the test piece to give the number of projections in a unit area of the topsheet to be measured.

The projecting-and-depressed pattern (the shape and the arrangement of projections) of the projecting-and-depressed region on the skin-facing surface of the topsheet 3A and 3B is not specifically limited, and any pattern may be appropriately selected without departing from the scope of the present invention.

Examples of the planar shape of the projection include a circular shape, an elliptical shape, a triangular shape, and a polygonal shape including a quadrangular shape. The projection may have a solid structure filled with constituent fibers of the topsheet therein or may have a hollow structure not filled with the constituent fibers.

An example of the projecting-and-depressed pattern of the projecting-and-depressed region is a pattern in which a plurality of projections are arranged in a scattered manner (for example, in a staggered manner), and depressions are arranged around the projections.

Another example of the projecting-and-depressed pattern of the projecting-and-depressed region is a pattern in which first linear depressions in plan view extending in a first direction intersecting both the longitudinal direction and the lateral direction and second linear depressions in plan view extending in a second direction intersecting the first direction are arranged in a grid pattern, and projections are present in a plurality of sections surrounded by the depressions.

Still another example of the projecting-and-depressed pattern of the projecting-and-depressed region is a pattern in which projections as ridges extending in the longitudinal direction or the lateral direction and depressions as grooves extending in the same direction are alternately arranged in the direction orthogonal to the extending direction.

Each non-skin-facing surface of the topsheets 3A and 3B is typically a flat face substantially having neither projections nor depressions as shown in FIG. 9(a) and FIG. 10(a).

The method of forming the projecting-and-depressed region of the topsheet 3A or 3B is not specifically limited. The projecting-and-depressed region is typically formed by subjecting an original fabric sheet as the material of the topsheet to partial compressing. In such a case, in compressed portions, a forming material of the original fabric sheet is compacted into depressions, whereas uncompressed portions project to one side in the thickness direction, specifically to the skin-facing surface side, into projections. In this case, the projections without compressing are low-density portions having a relatively low density, whereas the depressions with compressing are high-density portions having a relatively high density. For the compressing, any known method may be used, and examples include embossing with or without heat and ultrasonic embossing.

The topsheet 3A or 3B may have a single-layer structure or a laminate structure in which a plurality of layers are laminated in the thickness direction. An example of the topsheet having the laminate structure is a topsheet having a two-layer structure in which two sheets in a lamination state are partially joined, and one of the two sheets (skin-facing sheet) to be closer to the skin of a wearer projects in a direction away from the other sheet (non-skin-facing sheet) in portions other than the joined portions to form projections. As the sheet included in the topsheet, a nonwoven fabric is preferably used. Examples of the nonwoven fabric include air-through nonwoven fabric, heat roll nonwoven fabric, spunlace nonwoven fabric, spunbond nonwoven fabric, meltblown nonwoven fabric, and spunbond-meltblown-spunbond (SMS) nonwoven fabric.

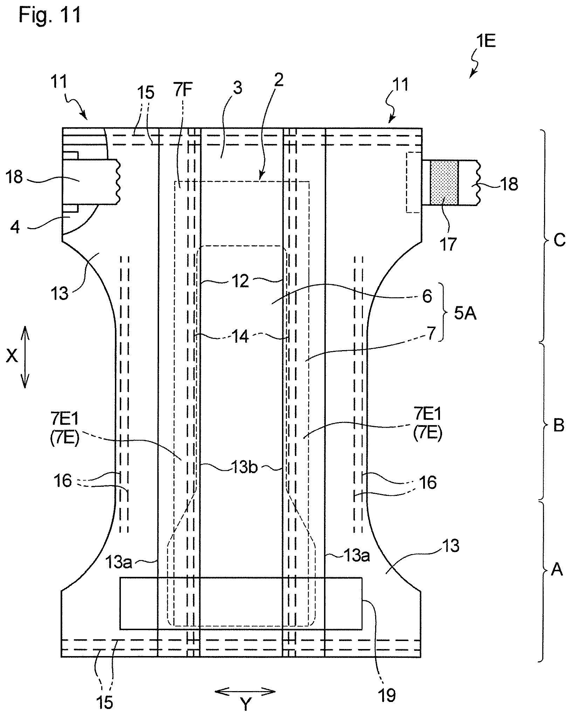

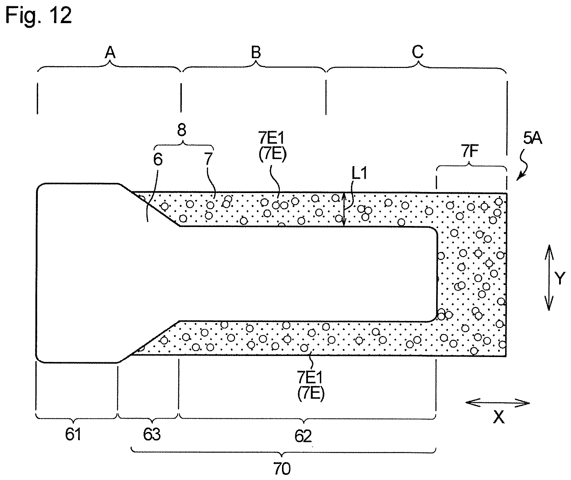

A diaper 1E shown in FIG. 11 differs from the above diaper 1 (see FIG. 1) in the absorbent member structure. FIG. 12 shows an absorbent member 5A included in the diaper 1E. The absorbent member 5A has a rectangular shape in plan view and extends in the longitudinal direction X from the front portion A to the rear portion C, and the length direction thereof is coincident with the longitudinal direction X. In the absorbent member 5A, the second layer 7 has a rectangular shape in plan view and has a constant dimension (width) in the lateral direction Y, whereas the first layer 6 has varying widths, which vary in the longitudinal direction X. Specifically, as shown in FIG. 12, the first layer 6 has, in the front portion A, a maximum width portion 61 having the maximum dimension (width) in the lateral direction Y in the first layer 6, has, in the rear portion C, a minimum width portion 62 having the minimum width in the first layer 6, and has, between the maximum width portion 61 and the minimum width portion 62, a variable width portion 63 having widths that gradually increase from the inside toward the outside in the longitudinal direction X (from the crotch portion B to the front portion A). The minimum width portion 62 is located at least in the crotch portion B and has a larger length in the longitudinal direction X than the maximum width portion 61 and the variable width portion 63. The first layer 6 has a smaller length in the longitudinal direction X than the second layer 7. On the side of the front portion A, the ends in the longitudinal direction X of the first layer 6 and the second layer 7 are coincident with each other, whereas the end in the longitudinal direction X of the first layer 6 on the side of the rear portion C is located inside in the longitudinal direction X from the end in the longitudinal direction X of the second layer 7 on the side of the rear portion C.

The second layer 7 extends outward from the peripheral edge of the first layer 6 in a portion other than the maximum width portion 61 (the minimum width portion 62 and the variable width portion 63), has lateral extensions 7E (crotch extensions 7E1), and has a longitudinal extension 7F that extends outward in the longitudinal direction X from the end in the longitudinal direction X of the first layer 6 in the rear portion C and from an imaginary extension line (not shown) of the end in the longitudinal direction X. The longitudinal extension 7F in the rear portion C and the lateral extensions 7E (crotch extensions 7E1) form an extension surrounding the first layer 6 in the rear portion C. In the embodiment shown in FIG. 11, the extension has a U shape in plan view.

In the diaper 1E, the second layer 7 has, in addition to the lateral extensions 7E (crotch extensions 7E1), the longitudinal extension 7F in the rear portion C, and thus the pressure on the legs of a wearer is reduced in the crotch portion B, as well as good cushioning properties in the rear portion C and excellent wearing comfort are achieved.

From the viewpoint of allowing the longitudinal extension 7F to more certainly achieve the advantageous effect, the length in the longitudinal direction X of the longitudinal extension 7F is preferably 2% or more and more preferably 5% or more and is preferably 50% or less and more preferably 40% or less relative to the length in the longitudinal direction X of the absorbent member 5A.

In the absorbent member 5A, as shown in FIG. 12, the side edges along the longitudinal direction X of the longitudinal end portion (maximum width portion 61) of the first layer 6 in the front portion A are located outside in the lateral direction Y from the side edges along the longitudinal direction X of the second layer 7 in a portion coincident in the longitudinal direction X with the longitudinal end portion (maximum width portion 61). In other words, in the longitudinal end portion in the front portion A, the first layer 6 protrudes in the lateral direction Y from the second layer 7. Such a structure improves the absorption capacity of the first layer 6, and consequently, the diaper 1E can have a higher liquid absorbability. A typical wearer of this kind of absorbent article (particularly, a disposable diaper) is likely to lie on the back while wearing the absorbent article, and thus it is important to improve the cushioning properties of the rear portion C as compared with the front portion A from the viewpoint of improving wearing comfort of the absorbent article. Hence, in the diaper 1E, the longitudinal extension 7F of the second layer 7 is provided in the rear portion C as described above to improve the cushioning properties of the rear portion C, whereas the first layer 6 in the front portion A has a larger width than the width of the second layer 7 to compensate a reduction in absorption capacity of the absorbent member 5A arising from the longitudinal extension 7F or a reduction in area of the first layer 6 in the rear portion C.

The present invention has been described on the basis of the preferred embodiments, but the absorbent article of the present invention is not limited to the above embodiments and can be appropriately modified. A portion only in one of the embodiments can be appropriately used exchangeably for another portion.

For example, from the viewpoint of improving absorbability, leak-proof performance, and the like, a liquid-permeable sheet including paper and various nonwoven fabrics, also called a second sheet, a sublayer sheet, or the like, may be interposed between the topsheet 3 and the absorbent member 5.

The absorbent article of the present invention is not limited to such an open type disposable diaper as in the above embodiments and widely encompasses articles used for absorption of body fluids excreted from a human body (such as urine, menstrual blood, loose feces, and sweat), and pull-on disposable diapers, sanitary napkins, sanitary shorts, and the like not having an attachment structure such as an attachment member 18 and an attachment region 19 are also encompassed.

### Industrial Applicability

According to the present invention, an absorbent article having excellent liquid absorbability, reducing the pressure on the legs of a wearer, and having excellent wearing comfort is provided.

## Claims

1. An absorbent article that has a longitudinal direction (X) extending from a front side through a crotch section (90) to a rear side of a wearer and a lateral direction (Y) orthogonal to the longitudinal direction (X) and is sectioned into a crotch portion (B) to be placed on the crotch section (90), a front portion (A) to be placed closer to the front side of a wearer than the crotch portion (B), and a rear portion (C) to be placed closer to the rear side of the wearer than the crotch portion (B),
the absorbent article comprising:
an absorbent member (5) to absorb and retain a body fluid; and
a topsheet (3) placed on a skin-facing surface side of the absorbent member (5),
wherein the absorbent member (5) has a laminate structure (8) in which a first layer (6) and a second layer (7) are laminated in a thickness direction,
in the first layer (6), a stack of a fiber material supports an absorbent polymer (P),
in the second layer (7), an absorbent polymer (P) is placed between two base sheets (71) placed to face together in the thickness direction,
the second layer (7) has, at least in the crotch portion (B), a crotch extension (7E1) extending outward in the lateral direction from a side edge, along the longitudinal direction (X), of the first layer (6) included in the laminate structure (8), when the crotch portion (B) is compressed in the lateral direction (Y) between legs of a wearer wearing the absorbent article, the crotch extension (7E1) affected by the compression is foldable along the side edge of the first layer (6),
at least in the crotch portion (B), in a region coincident in the longitudinal direction (X) with the crotch extension (7E1), the first layer (6) has a larger absorbent polymer basis weight than the second layer (7), and the second layer (7) has a larger absorbent polymer density than the first layer (6), and
the absorbent polymer basis weight of the second layer (7) in a portion included in the laminate structure (8) is larger than that of the crotch extension (7E1).

2. The absorbent article according to claim 1, wherein a lateral dimension of the crotch extension (7E1) is larger than a thickness of the first layer (6) in a portion coincident in the longitudinal direction (X) with the crotch extension (7E1).

3. The absorbent article according to claim 2, wherein a region satisfying magnitude relation of the lateral dimension (Y) of the crotch extension (7E1) > the thickness of the first layer (6) in a portion coincident in the longitudinal direction (X) with the crotch extension (7E1) is present in the crotch portion (B) in 50% or more of an entire length in the longitudinal direction (X) of the crotch portion (B).

4. The absorbent article according to any one of claims 1 to 3, wherein the second layer (7) is placed on a non-skin-facing surface side of the first layer (6).

5. The absorbent article according to any one of claims 1 to 4, wherein the crotch extension (7E1) is foldable to rise toward the crotch section (90) of a wearer wearing the absorbent article when the crotch portion (B) is compressed in the lateral direction (Y) between legs (91) of the wearer.

6. The absorbent article according to claim 5, wherein the topsheet (3) extends in the lateral direction (Y) from the laminate structure (8) to the crotch extension (7E1), a projecting-and-depressed region including a plurality of projections (30) projecting toward a skin of a wearer is formed on a skin-facing surface of the topsheet (3), and
when the crotch extension (7E1) is folded to rise toward the crotch section (90) of a wearer wearing the absorbent article, the projections (30) of the topsheet (3) are present between the rising crotch extension (7E1) and the first layer (6).

7. The absorbent article according to claim 6, wherein the projecting-and-depressed region of the topsheet (3) has a first region (32) overlapping with the laminate structure (8) in plan view and a second region (33) overlapping with the crotch extension (7E1) in plan view,
a projection (30A) in the first region (32) has a larger projecting height or a larger area than a projection (30B) in the second region (33), and when the crotch extension (7E1) is folded to rise toward the crotch section (90) of a wearer wearing the absorbent article, the projection (30B) in the second region (33) is present between the rising crotch extension (7E1) and the first layer (6).

8. The absorbent article according to any one of claims 1 to 7, wherein the second layer (7) has a nook extension (7E2) that is located at a nook portion (50) of the absorbent member (5) and is not connected to the crotch extension (7E1).

9. The absorbent article according to claim 8, wherein in a region coincident in the longitudinal direction (X) with the nook extension (7E2), the first layer (6) has a larger absorbent polymer basis weight than the second layer (7), and the second layer (7) has a larger absorbent polymer density than the first layer (6).

10. The absorbent article according to claim 8 or 9, wherein a lateral dimension of the nook extension (7E2) is larger than a thickness of the first layer (6) in a portion coincident in the longitudinal direction (X) with the nook extension (7E2).

11. The absorbent article according to any one of claims 1 to 7, wherein the second layer (7) has a longitudinal extension (7F) extending outward in the longitudinal direction (X) from a longitudinal end of the first layer (6) in the rear portion (C) and from an imaginary extension line of the longitudinal end, and the longitudinal extension (7F) and the crotch extension (7E1) form an extension surrounding the rear portion of the first layer (6).

12. The absorbent article according to claim 11, wherein the extension has a U shape in plan view.

13. The absorbent article according to claim 11 or 12, wherein a longitudinal length of the longitudinal extension (7F) is 2% or more and 50% or less relative to a longitudinal length of the absorbent member (5).

14. The absorbent article according to any one of claims 1 to 13, wherein a side edge along the longitudinal direction (X) in a longitudinal end portion of the first layer (6) in the front portion (A) is located outside in the lateral direction from a side edge along the longitudinal direction (X) of the second layer (7) in a portion coincident in the longitudinal direction (X) with the longitudinal end portion.

15. The absorbent article according to any one of claims 1 to 14, wherein an absorbent polymer placement layer of the second layer (7) contains a smaller amount of an absorbent fiber material than the first layer (6) or contains no absorbent fiber material.

## Patentansprüche

1. Saugfähiger Artikel, der eine Längsrichtung (X), die sich von einer Vorderseite durch einen Schrittbereich (90) zu einer Rückseite eines Trägers erstreckt, und eine Querrichtung (Y) orthogonal zu der Längsrichtung (X) aufweist und in einen Schrittabschnitt (B), der auf dem Schrittbereich (90) zu platzieren ist, einen Vorderabschnitt (A), der näher an der Vorderseite eines Trägers als der Schrittabschnitt (B) zu platzieren ist, und einen Rückabschnitt (C), der näher an der Rückseite des Trägers als der Schrittabschnitt (B) zu platzieren ist, unterteilt ist,
der saugfähige Artikel aufweisend:
ein saugfähiges Element (5), um eine Körperflüssigkeit aufzusaugen und zu halten; und
eine Decklage (3), die auf einer der Haut zugewandten Oberflächenseite des saugfähigen Elements (5) platziert ist,
wobei das saugfähige Element (5) eine Laminatstruktur (8) aufweist, in der eine erste Schicht (6) und eine zweite Schicht (7) in einer Dickenrichtung laminiert sind,
in der ersten Schicht (6) ein Stapel eines Fasermaterials ein saugfähiges Polymer (P) lagert,
in der zweiten Schicht (7) ein saugfähiges Polymer (P) zwischen zwei Basislagen (71) platziert ist, die in der Dickenrichtung einander zugewandt platziert sind,
die zweite Schicht (7) mindestens in dem Schrittabschnitt (B) eine Schritterweiterung (7E1) aufweist, die sich in der Querrichtung von einer Seitenkante entlang der Längsrichtung (X) der ersten Schicht (6), die in der Laminatstruktur (8) eingeschlossen ist, nach außen erstreckt, wobei, wenn der Schrittabschnitt (B) in der Querrichtung (Y) zwischen den Beinen eines Trägers zusammengedrückt wird, der den saugfähigen Artikel trägt, die Schritterweiterung (7E1), die von dem Zusammendrücken betroffen ist, entlang der Seitenkante der ersten Schicht (6) faltbar ist,
mindestens in dem Schrittabschnitt (B), in einem Bereich, der in der Längsrichtung (X) mit der Schritterweiterung (7E1) zusammenfällt, die erste Schicht (6) ein größeres Flächengewicht von saugfähigem Polymer als die zweite Schicht (7) aufweist und die zweite Schicht (7) eine größere Dichte von saugfähigem Polymer als die erste Schicht (6) aufweist, und
das Flächengewicht von saugfähigem Polymer der zweiten Schicht (7) in einem Abschnitt, der in der Laminatstruktur (8) eingeschlossen ist, größer als das der Schritterweiterung (7E1) ist.

2. Saugfähiger Artikel nach Anspruch 1, wobei eine Querabmessung der Schritterweiterung (7E1) größer als eine Dicke der ersten Schicht (6) in einem Abschnitt ist, der in der Längsrichtung (X) mit der Schritterweiterung (7E1) zusammenfällt.

3. Saugfähiger Artikel nach Anspruch 2, wobei ein Bereich, der ein Größenverhältnis der Querabmessung (Y) der Schritterweiterung (7E1) > die Dicke der ersten Schicht (6) in einem Abschnitt erfüllt, der in der Längsrichtung (X) mit der Schritterweiterung (7E1) zusammenfällt, in dem Schrittabschnitt (B) in 50 % oder mehr einer Gesamtlänge in der Längsrichtung (X) des Schrittabschnitts (B) vorhanden ist.

4. Saugfähiger Artikel nach einem der Ansprüche 1 bis 3, wobei die zweite Schicht (7) auf einer nicht der Haut zugewandten Oberflächenseite der ersten Schicht (6) platziert ist.

5. Saugfähiger Artikel nach einem der Ansprüche 1 bis 4, wobei die Schritterweiterung (7E1) faltbar ist, um sich in Richtung des Schrittabschnitts (90) eines Trägers zu erheben, der den saugfähigen Artikel trägt, wenn der Schrittbereich (B) in der Querrichtung (Y) zwischen den Beinen (91) des Trägers zusammengedrückt wird.

6. Saugfähiger Artikel nach Anspruch 5, wobei sich die Decklage (3) in der Querrichtung (Y) von der Laminatstruktur (8) zu der Schritterweiterung (7E1) erstreckt, ein vorspringender und vertiefter Bereich, der mehrere von in Richtung einer Haut eines Trägers vorspringenden Vorsprüngen (30) einschließt, auf einer der Haut zugewandten Oberfläche der Decklage (3) ausgebildet ist, und
wenn die Schritterweiterung (7E1) gefaltet ist, um sich in Richtung des Schrittabschnitts (90) eines Trägers, der den saugfähigen Artikel trägt, zu erheben, die Vorsprünge (30) der Decklage (3) zwischen der sich erhebenden Schritterweiterung (7E1) und der ersten Schicht (6) vorhanden sind.

7. Saugfähiger Artikel nach Anspruch 6, wobei der vorspringende und vertiefte Bereich der Decklage (3) einen in Draufsicht mit der Laminatstruktur (8) überlappenden ersten Bereich (32) und einen in Draufsicht mit der Schritterweiterung (7E1) überlappenden zweiten Bereich (33) aufweist,
ein Vorsprung (30A) in dem ersten Bereich (32) eine größere vorspringende Höhe oder eine größere Fläche als ein Vorsprung (30B) in dem zweiten Bereich (33) aufweist, und wenn die Schritterweiterung (7E1) gefaltet wird, um sich in Richtung des Schrittabschnitts (90) eines Trägers zu erheben, der den saugfähigen Artikel trägt, der Vorsprung (30B) in dem zweiten Bereich (33) zwischen der sich erhebenden Schritterweiterung (7E1) und der ersten Schicht (6) vorhanden ist.

8. Saugfähiger Artikel nach einem der Ansprüche 1 bis 7, wobei die zweite Schicht (7) eine Eckenerweiterung (7E2) aufweist, die sich an einem Eckenabschnitt (50) des saugfähigen Elements (5) befindet und nicht mit der Schritterweiterung (7E1) verbunden ist.

9. Saugfähiger Artikel nach Anspruch 8, wobei in einem Bereich, der in der Längsrichtung (X) mit der Eckenerweiterung (7E2) zusammenfällt, die erste Schicht (6) ein größeres Flächengewicht an saugfähigem Polymer als die zweite Schicht (7) aufweist und die zweite Schicht (7) eine größere Dichte an saugfähigem Polymer als die erste Schicht (6) aufweist.

10. Saugfähiger Artikel nach Anspruch 8 oder 9, wobei eine Querabmessung der Eckenerweiterung (7E2) größer als eine Dicke der ersten Schicht (6) in einem Abschnitt ist, der in der Längsrichtung (X) mit der Eckenerweiterung (7E2) zusammenfällt.

11. Saugfähiger Artikel nach einem der Ansprüche 1 bis 7, wobei die zweite Schicht (7) eine Längserweiterung (7F) aufweist, die sich in der Längsrichtung (X) von einem Längsende der ersten Schicht (6) in dem Rückabschnitt (C) und von einer imaginären Erweiterungslinie des Längsendes nach außen erstreckt, und die Längserweiterung (7F) und die Schritterweiterung (7E1) eine Erweiterung ausbilden, die den Rückabschnitt der ersten Schicht (6) umgibt.

12. Saugfähiger Artikel nach Anspruch 11, wobei die Erweiterung in Draufsicht eine U-Form besitzt.

13. Saugfähiger Artikel nach Anspruch 11 oder 12, wobei eine Längslänge der Längserweiterung (7F) 2 % oder mehr und 50 % oder weniger bezogen auf eine Längslänge des saugfähigen Elements (5) beträgt.

14. Saugfähiger Artikel nach einem der Ansprüche 1 bis 13, wobei sich eine Seitenkante entlang der Längsrichtung (X) in einem Längsendabschnitt der ersten Schicht (6) in dem Vorderabschnitt (A) in der Querrichtung außerhalb einer Seitenkante entlang der Längsrichtung (X) der zweiten Schicht (7) in einem Abschnitt befindet, der in der Längsrichtung (X) mit dem Längsendabschnitt zusammenfällt.

15. Saugfähiger Artikel nach einem der Ansprüche 1 bis 14, wobei eine saugfähige Polymerplatzierungsschicht der zweiten Schicht (7) eine geringere Menge eines saugfähigen Fasermaterials als die erste Schicht (6) enthält oder kein saugfähiges Fasermaterial enthält.

## Revendications

1. Article absorbant qui a une direction longitudinale (X) s'étendant depuis un côté avant, à travers une section d'entrejambe (90), jusqu'à un côté arrière d'un porteur et une direction latérale (Y) orthogonale à la direction longitudinale (X) et est divisé en une partie d'entrejambe (B) destinée à être placée sur la section d'entrejambe (90), une partie avant (A) destinée à être placée plus près du côté avant d'un porteur que la partie d'entrejambe (B), et une partie arrière (C) destinée à être placée plus près du côté arrière du porteur que la partie d'entrejambe (B),
l'article absorbant comprenant :
un élément absorbant (5) pour absorber et retenir un fluide corporel ; et
une feuille supérieure (3) placée sur un côté de surface orientée vers la peau de l'élément absorbant (5),
dans lequel l'élément absorbant (5) a une structure stratifiée (8) dans laquelle une première couche (6) et une seconde couche (7) sont stratifiées dans une direction de l'épaisseur,
dans la première couche (6), un empilement d'un matériau fibreux soutient un polymère absorbant (P),
dans la seconde couche (7), un polymère absorbant (P) est placé entre deux feuilles de base (71) placées face à face dans la direction de l'épaisseur,
la seconde couche (7) a, au moins dans la partie d'entrejambe (B), une extension d'entrejambe (7E1) s'étendant vers l'extérieur dans la direction latérale depuis un bord latéral, le long de la direction longitudinale (X), de la première couche (6) incluse dans la structure stratifiée (8), lorsque la partie d'entrejambe (B) est comprimée dans la direction latérale (Y) entre les jambes d'un porteur portant l'article absorbant, l'extension d'entrejambe (7E1) concernée par la compression est pliable le long du bord latéral de la première couche (6),
au moins dans la partie d'entrejambe (B), dans une région coïncidant dans la direction longitudinale (X) avec l'extension d'entrejambe (7E1), la première couche (6) a une masse surfacique de polymère absorbant plus importante que celle de la seconde couche (7), et la seconde couche (7) a une densité de polymère absorbant plus importante que celle de la première couche (6), et
la masse surfacique de polymère absorbant de la seconde couche (7) dans une partie incluse dans la structure stratifiée (8) est supérieure à celle de l'extension d'entrejambe (7E1).

2. Article absorbant selon la revendication 1, dans lequel une dimension latérale de l'extension d'entrejambe (7E1) est supérieure à une épaisseur de la première couche (6) dans une partie coïncidant dans la direction longitudinale (X) avec l'extension d'entrejambe (7E1).

3. Article absorbant selon la revendication 2, dans lequel une région satisfaisant à une relation de grandeur de la dimension latérale (Y) de l'extension d'entrejambe (7E1) > l'épaisseur de la première couche (6) dans une partie coïncidant dans la direction longitudinale (X) avec l'extension d'entrejambe (7E1) est présente dans la partie d'entrejambe (B) sur 50 % ou plus d'une longueur totale dans la direction longitudinale (X) de la partie d'entrejambe (B).

4. Article absorbant selon l'une quelconque des revendications 1 à 3, dans lequel la seconde couche (7) est placée sur un côté de surface non orientée vers la peau de la première couche (6).

5. Article absorbant selon l'une quelconque des revendications 1 à 4, dans lequel l'extension d'entrejambe (7E1) est pliable pour s'élever vers la section d'entrejambe (90) d'un porteur portant l'article absorbant lorsque la partie d'entrejambe (B) est comprimée dans la direction latérale (Y) entre les jambes (91) du porteur.

6. Article absorbant selon la revendication 5, dans lequel la feuille supérieure (3) s'étend dans la direction latérale (Y) depuis la structure stratifiée (8) jusqu'à l'extension d'entrejambe (7E1), une région en saillie et en creux comportant une pluralité de saillies (30) faisant saillie vers la peau d'un porteur est formée sur une surface orientée vers la peau de la feuille supérieure (3), et
lorsque l'extension d'entrejambe (7E1) est pliée pour s'élever vers la section d'entrejambe (90) d'un porteur portant l'article absorbant, les saillies (30) de la feuille supérieure (3) sont présentes entre l'extension d'entrejambe (7E1) qui s'élève et la première couche (6).

7. Article absorbant selon la revendication 6, dans lequel la région en saillie et en creux de la feuille supérieure (3) a une première région (32) chevauchant la structure stratifiée (8) en vue en plan et une seconde région (33) chevauchant l'extension d'entrejambe (7E1) en vue en plan,
une saillie (30A) dans la première région (32) a une hauteur de saillie plus grande ou une surface plus grande que celle d'une saillie (30B) dans la seconde région (33), et lorsque l'extension d'entrejambe (7E1) est pliée pour s'élever vers la section d'entrejambe (90) d'un porteur portant l'article absorbant, la saillie (30B) dans la seconde région (33) est présente entre l'extension d'entrejambe (7E1) qui s'élève et la première couche (6).

8. Article absorbant selon l'une quelconque des revendications 1 à 7, dans lequel la seconde couche (7) présente une extension de coin (7E2) qui est située au niveau d'une partie formant coin (50) de l'élément absorbant (5) et n'est pas reliée à l'extension d'entrejambe (7E1).

9. Article absorbant selon la revendication 8, dans lequel, dans une région coïncidant dans la direction longitudinale (X) avec l'extension de coin (7E2), la première couche (6) a une masse surfacique de polymère absorbant plus importante que celle de la seconde couche (7), et la seconde couche (7) présente une densité de polymère absorbant plus importante que celle de la première couche (6).

10. Article absorbant selon la revendication 8 ou 9, dans lequel une dimension latérale de l'extension de coin (7E2) est supérieure à une épaisseur de la première couche (6) dans une partie coïncidant dans la direction longitudinale (X) avec l'extension de coin (7E2).

11. Article absorbant selon l'une quelconque des revendications 1 à 7, dans lequel la seconde couche (7) a une extension longitudinale (7F) s'étendant vers l'extérieur dans la direction longitudinale (X) à partir d'une extrémité longitudinale de la première couche (6) dans la partie arrière (C) et à partir d'une ligne d'extension imaginaire de l'extrémité longitudinale, et l'extension longitudinale (7F) et l'extension d'entrejambe (7E1) forment une extension entourant la partie arrière de la première couche (6).

12. Article absorbant selon la revendication 11, dans lequel l'extension a une forme en U en vue en plan.

13. Article absorbant selon la revendication 11 ou 12, dans lequel une longueur longitudinale de l'extension longitudinale (7F) est de 2 % ou plus et de 50 % ou moins par rapport à une longueur longitudinale de l'élément absorbant (5).

14. Article absorbant selon l'une quelconque des revendications 1 à 13, dans lequel un bord latéral le long de la direction longitudinale (X) dans une partie d'extrémité longitudinale de la première couche (6) dans la partie avant (A) est situé à l'extérieur dans la direction latérale par rapport à un bord latéral le long de la direction longitudinale (X) de la seconde couche (7) dans une partie coïncidant dans la direction longitudinale (X) avec la partie d'extrémité longitudinale.

15. Article absorbant selon l'une quelconque des revendications 1 à 14, dans lequel une couche de placement de polymère absorbant de la seconde couche (7) contient une plus petite quantité d'un matériau fibreux absorbant que la première couche (6) ou ne contient aucun matériau fibreux absorbant.
